# EUROPEAN PATENT SPECIFICATION

(11) **EP 1 616 877 B1**
(45) Date of publication and mention of the grant of the patent: **21.05.2008**
(21) Application number: 04016656.3
(22) Date of filing: 15.07.2004
(51) Int. Cl.: C07K 14/415, G01N 33/68, A61K 38/16, A61K 39/35

(54) **Reagents for diagnosis and therapy of latex allergy and method for the preparation of the same**
Reagenzien zur Diagnose und Behandlung von Latexallergie und Verfahren zu ihrer Herstellung
Réactifs pour le diagnostic et le traitement de l'allergie au latex et procédé de leur production

(43) Date of publication of application: 18.01.2006
(73) Proprietor: BIOMAY AG, 1090 Wien (AT)
(72) Inventor: Wagner, Stefan, 1070 Wien (AT); Breiteneder, Heimo, 1070 Wien (AT)
(74) Representative: Keller, Günter

(56) References cited:
- EP-A- 0 704 457
- WO-A-01/61305
- WAGNER S ET AL.: "Hev b 9, an enolase and a new cross-reactive allergen from Hevea latex and molds" EUROPEAN JOURNAL OF BIOCHEMISTRY, vol. 267, 2000, pages 7006-7014, XP002330929
- NIETO A ET AL.: "Assessment of profilin as an allergen for latex-sensitized patients" ALLERGY, vol. 57, 2002, pages 776-784, XP002330930
- ZHIPING CHEN ET AL: "Isolation and identification of hevein as a major IgE-binding polypeptide in Hevea latex" JOURNAL OF ALLERGY AND CLINICAL IMMUNOLOGY, MOSBY - YEARLY BOOK, INC, US, vol. 99, no. 3, 1997, pages 402-409, XP002957470 ISSN: 0091-6749
- ALENIUS H ET AL: "THE MAIN IGE-BINDING EPITOPE OF A MAJOR LATEX ALLERGEN, PROHEVEIN, IS PRESENT IN ITS N-TERMINAL 43-AMINO ACID FRAGMENT, HEVEIN" JOURNAL OF IMMUNOLOGY, THE WILLIAMS AND WILKINS CO. BALTIMORE, US, vol. 156, no. 4, 1996, pages 1618-1625, XP001152539 ISSN: 0022-1767
- DATABASE BIOSIS BIOSCIENCES INFORMATION SERVICE, PHILADELPHIA, PA, US; 1994, ALENIUS H ET AL.: "Latex allergy: Frequent occurrence of IgE antibodies to a cluster of 11 latex proteins in patients with spina bifida and histories of anaphylaxis" XP002330943 Database accession no. PREV199497322640 & JOURNAL OF LABORATORY AND CLINICAL MEDICINE, vol. 123, no. 5, 1994, pages 712-720, ISSN: 0022-2143
- HUFNAGL K ET AL.: "Induction of mucosal tolerance with recombinant Hev b 1 and recombinant Hev b 3 for prevention of latex allergy in BALB/c mice" CLINICAL AND EXPERIMENTAL IMMUNOLOGY, vol. 133, 2003, pages 170-176, XP002330931
- SANCHEZ-MONGE R ET AL.: "Isolation and characterization of major banana allergens: identification as fruit class I chitinases" CLINICAL AND EXPERIMENTAL ALLERGY, vol. 29, 1999, pages 673-680, XP002330932

## Description

The present invention relates to reagents for diagnosis and/or therapy of latex allergy or fruit allergy and to a method for the preparation of the reagents.

Type I allergy to natural rubber latex (NRL) has emerged during the last two decades of the 20th century as an increasing health problem with far-reaching consequences for patients, regarding both their occupational situation and safety in medical care. Latex "sensitization" or "sensitivity" is defined as the presence of specific IgE antibodies to latex allergens in an individual's serum. Latex "allergy" describes the elicitation of immediate-type allergic symptoms by contact with latex or latex products in a sensitized individual.

Most of the natural rubber in the world is obtained from the latex of the rubber tree *Hevea brasiliensis.* The composition of natural rubber latex is rather complex and it contains a variety of substances like water, rubber (cis-1,4-polyisoprene), proteins, resins, sugars, ash, and sterol glycosides. For commercial rubber products, fresh latex is mixed with ammonia to a final concentration of 200 mmol/L to avoid coagulation and then termed ammoniated latex (AL). On the other hand, high-speed centrifugation of non-ammoniated latex (NAL) yields three different fractions: (i) a white creamy layer of rubber particles at the top, (ii) a bottom fraction (B-serum) that consists mainly of the vacuole-like lutoids and (iii) a yellowish C-serum between containing the cytosol from the cells of the latex vessels.

The first step in the diagnosis of type I allergy to NRL is to record a comprehensive clinical history of allergic symptoms to NRL. In a second step, necessary confirmatory tests are performed to identify a state of sensitization. The most commonly used confirmatory tests are the skin prick test (SPT) and the serum test for latex-specific IgE antibodies. Standardized SPT extracts and well-characterized allergen mixtures for IgE determination are therefore important prerequisites to diagnose a latex allergy.

The detection of allergen-specific IgE antibodies in the skin is an attractive method because it is rapid, sensitive, and involves a clinically observable and biological relevant response in the skin or the individual. In 1995 and 1996 skin prick tests were performed with the Bencard latex reagent that was based on an unprocessed AL produced in Canada. The investigators described a 4.2% prevalence of positive latex skin test responses among hospitalized patients in an asthma and allergy practice¹ and a positive association between the size of the SPT response and the severity of the latex-induced symptoms². In an initial phase I/II clinical study the relative safety and diagnostic accuracy of three candidate latex source materials - AL, NAL, and extracts of powdered latex gloves - was determined. As a result, NAL was described as the most attractive candidate latex for further development because of its stability and ease of quality control³. Ebo et al. (J Allergy Clin Immunol. 1997 Nov; 100(5):618-23) carried out a comparative study of IgE antibody detection methods including SPT. They used an NAL extract (Diagnostic Products Corp) that showed a sensitivity of 68% and a specificity of 100%. In a Finish study the diagnostic performance of a SPT reagent based on latex C-serum of NAL from Stallergènes (Fresnes, France) was examined⁴. A diagnostic sensitivity of 93% and a specificity of 100% was reported. This is the only standardized commercial natural rubber latex extract available today in Europe. A multicenter phase III clinical study was performed with the Investigational Greer reagent (Greer Laboratories, Lenoir, NC), with an NAL based extract ⁵. In this study the diagnostic sensitivity was 95% and the specificity 100%. Reanalyzing of the data changed the overall sensitivity of the Greer NAL SPT reagent to 70% - 75% ⁶.

Furthermore, an additional SPT extract is available from ALK-Abello (Hörsholm, Denmark), which is based on latex C-serum of NAL available. Besides commercially available products, allergists who wish to perform diagnostic skin tests may prepare their own in-house latex extracts from gloves, especially in case in the respective country, e.g. in the USA, there is no standardized latex skin testing reagent licensed by the corresponding drug approval organisation (e.g. FDA).

The most frequent techniques for *in vitro* measurement of natural latex-specific IgE in a serum are the radioallergosorbent assay (RAST) and the enzyme-linked immunosorbent assay (ELISA). Several radioallergosorbent tests are commercially available. Three in vitro assays (DPC Microplate Alastat, Pharmacia CAP system FEIA, and Hycor HYTECH) were compared for the detection of natural rubber latex-specific IgE antibody ⁷. The Pharmacia CAP System and DPC's Alastat displayed a diagnostic specificity of 97% and a 76 or 73% diagnostic sensitivity, respectively, when compared with the latex skin test with the Greer experimental latex reagent. Both assays misclassified approximately 25% of latex-sensitized cases as falsely negative for latex-specific IgE antibodies. In a separate study of performance the diagnostic sensitivity and specificity displayed by both assays were equivalent to those in the above mentioned study ⁸. The HYTECH assay in the first study displayed a specificity of 73%, which indicates that it produced 27% false-positive results when compared with the skin test ⁷. It has been speculated that the reasons for the low diagnostic sensitivity in the CAP and AlaSTAT assays may be related to poorly represented and/or denatured allergens in the allergen-containing reagents used in these assays ⁶. It was concluded that the accuracy and reliability of assays employing reagents derived from whole natural rubber latex depend on the availability of the allergens and their presence in molar excess in relation to IgE antibody.

The most widely used RAST kit is probably the latex RAST k82 produced by Pharmacia Diagnostics and its enzyme-linked immunosorbant assay (Pharmacia CAP system). A further new latex RAST from Pharmacia Diagnostics is based on k82 but spiked with a recombinant Hev b 5 (rHev b 5) molecule. Additionally, RASTs based on recombinant latex allergens are available. Recombinant Hev b 1, 2, 3, 6.01, 6.02, 8, 9, and 11 are available as fusion proteins with the maltose binding protein (MBP), rHev b 5 without MBP.

In 2000, Yip et al. showed that recombinant latex allergens are clinically reactive, can be produced in standardized manner, and could potentially provide sensitive and specific reagents for the diagnosis of latex allergy ⁹. Nevertheless, the diagnostic capacity of a recombinant protein depends on the equivalence to the natural protein. SPTs with natural Hev b 2 showed that this allergen is one of the major latex allergens ¹⁰. Raulf Heimsoth et al. described a poor correlation between the IgE-reactivity of rHev b 2 and natural Hev b 2 ¹¹. They observed almost no IgE-reactivity to their rHev b 2. This observation may be due to the wrong folding of the the *E*. *coli* product or to carbohydrate residues of Hev b 2 that represent IgE binding epitopes ¹² and are not present on a recombinant protein expressed in *E*. *coli*.

European patent application EP 0 704 457 discloses the purification of only three individual latex allergens designated Hev b II, III and IV. The three allergens disclosed in more detail represent only a very small part of the numerous latex allergens and do in particular not even include the most important latex allergens now designated for example Hev b 5.

The international patent application WO 01/61305 discloses the use of monoclonal and polyclonal antibodies which are directed against only four recombinant latex allergens, namely Hev b 1, 3, 5, 6.02 and its use in immunological methods.

The publication Wagner et al., Eur.J.Biochem. 267 (2000), pp. 7006-7014 describes a method which was developed in order to purify one single allergen, namely Hev b 9.

Furthermore, the publication of Hufnagel et al., Clin. Exp. Immunol. 133 (2003), pp. 170 - 176 discloses a mouse model of latex allergy using two major latex allergens for HCWs (health-care workers) and SB (spina bifida) patients, Hev b 1 and Hev b 3, for sensitization.

To date, thirteen latex allergens have been recognized by the International Union of Immunological Societies. It is reported that Natural rubber latex from the *Hevea brasiliensis* tree contains more than 250 polypeptides, at least 60 of which demonstrate IgE binding properties ¹³. Therefore, diagnosis of latex allergy with the 13 recombinant or purified allergens seems not to be sufficient. Additionally, some of the major latex allergens originate from the B-serum. However, all of the commercially available latex extracts derived from NAL are exclusively derived from latex C-serum.

Fresh latex contains about 1- 2 proteins and can be separated by high speed centrifugation into three main fractions: (i) a white upper layer or rubber particles; (ii) an aqueous layer (C-serum) containing the cytoplasm from the cells of the latex vessels; and (iii) the bottom fraction (B-serum), which consists mainly of the vacuole-like lutoids).

Furthermore, a comparison of commercially available testing procedures for latex allergy revealed a wide varation of the results and no method under assessment could be correlated with reported symptoms of type I latex allergy¹⁸. A major disadvantage of the currently available tests is that todate several in vitro methods are necessary to detect IgE sensitisation to latex¹⁹.

Therefore, a technical problem underlying the present invention is to provide improved reagents for diagnosis and therapy of latex and related allergies.

The solution to the above technical problem is provided by the embodiments characterised in the claims.

In particular, the present invention provides a method for the production of a composition containing substantially all natural human IgE binding proteins of natural rubber latex (NRL) comprising the step of preparing one or more protein fraction(s) from NRL or one or more exctract(s) thereof by fractionated precipitation and/or chromatographic separation.

The term "composition" as referred to herein generally comprises one or more protein fractions derived from NRL (or any extract or fraction thereof).

The term "natural rubber latex" means all rubber latexes obtainable from any natural source. It has been observed that in raw latex the amount of proteins and their molecular weight distribution vary considerably among different sources of raw material attributed to factors such as the origin, growing and maturation conditions (climate, season, soil) ^{14,15}. However, in case NRL is collected from a particular clone of the *Hevea brasiliensis* tree (e.g. *Hevea brasiliensis* Rubber Research Institute of Malaysia clone RRIM 600), by means of a standardized preparation procedure, the protein content and specificity are uniform and reproducible ¹⁶. Therefore, it is preferred that the NRL is from *H. brasiliensis.* Preferably, the NRL is non-ammoniated latex (NAL).

The term "natural human IgE binding proteins of natural rubber latex" means all proteins present in NRL that can act as an allergen in humans and thus contribute to latex allergy or at least sensitisation.

The expression "extract of NRL" comprises any fraction or modified solution or suspension or emulsion derived from NRL as long as said extract contains all natural latex allergens according to the above definition.

Particularly preferred extracts of NRL, in particular NAL, are obtained by centrifugation, preferably high speed centrifugation yielding the above-described fractions (i) white creamy layer of rubber particles, (ii) B-serum (bottom) and (iii) C - serum between (i) and (ii). For the purposes of the present invention preferred extracts of NAL are the B-serum and the C-serum.

In one embodiment of the present invention, fractionating different batches of NRL collected by a standardized preparation procedure yields protein extracts of very similar composition.

Thus, the method according to the present invention, comprises the steps of
(a) preparing one or more protein fraction(s) of NRL or an extract thereof by fractionated precipitation and
(b) subjecting the protein fraction(s) obtained in step (a) to chromatographic separation.

The term "fractionated precipitation" means that proteins can be precipitated by causing perturbations in the solvent with respect to pH, ionic strength, and temperature. The precipitate is preferably recovered by commonly used centrifugation steps. The properties of the solvent can also be modified by addition of high concentrations of certain salts or of miscible organic solvents. Salts in solution at low ionic strength relative to that of isotonic saline may represent a perturbation that can cause certain proteins to precipitate from solution. On the other hand, salts present in very high concentrations with ionic strength much greater than that of tissue media will cause the precipitation of many proteins. Precipitation occurs by neutralization of surface charges by the salt, by reducing the chemical activity of the protein, and by diminishing the effective concentration of the water. This is called "salting out" of proteins. The concentration of any salt necessary to cause precipitation of a particular protein is related to the number and distribution of charges and of nonionic polar groups on the surface of the protein, and to the number and distribution of hydrophobic residues exposed and rendered dominant as the charges are neutralized. The size and shape of the protein also contribute to the relative ease of precipitability. Ammonium sulfate is the precipitant used most frequently in the salting out of proteins. Its major advantages are (1) at saturation, it is of sufficiently high molarity that it causes the precipitation of most proteins; (2) it does not have a large heat of solution; (3) even its saturated solution has a density that is not so large that it interferes with the sedimentation of most precipitated proteins by centrifugation; (4) its concentrated solutions prevent or limit most bacterial growth; and (5) in solution it protects most proteins from denaturation.

It is therefore part of the present invention that, in a first step, proteins of latex C- or B-serum are fractionated by different concentrations of ammonium sulfate.

In a preferred embodiment of the present invention, proteins of the fractions are further separated by ion exchange chromatography as a second step.

Therefore, the method of the present invention preferably comprises the steps of:
(i) preparing one or more protein fraction(s) of NAL C-serum by fractionated precipitation,
(ii) subjecting one or more of the protein fraction(s) obtained in step (a) to chromatographic separation, and
(iii) subjecting NAL B-serum to chromatographic separation.

The chromatographic separation may include ion-exchange chromatography, size exclusion chromatography, hydrophobic chromatography and/or by affinity chromatography, preferable using latex allergen-specific immunoglobulins.

Ion-exchange chromatography (IEC) is the preferred chromatographic technique of the present invention. IEC is designed specifically for the separation of ionic or ionisable compounds. IEC differs from other types of liquid chromatography in that the stationary phase carries ionisable functional groups, fixed by chemical bonding to the stationary phase. To satisfy requirements for electrical neutrality, these fixed charges will carry a counterion of opposite sign. This counterion is not fixed and can be displaced. Two separate events are involved in IEC. First the binding of the protein to the fixed charges and second the elution or displacement of the protein from the fixed charges by a new counterion with a greater affinity for the fixed charges than the protein. Preferably, anion-exchange chromatography is used for the chromatographic separation of NRL exctracts or protein fractions thereof, e.g. NAL, NAL C- or B-serum or precipitates obtained by fractionated precipitation, preferably salting out using ammonium sulphate, of NRL (e.g. NAL), NAL B- or C-serum.

In a preferred embodiment MonoBeads (Pharmacia, Uppsala, Sweden) are used as ion media, which are based on 10 µm beaded hydrophilic polystyrene/divinyl benzene resin. Preferably, the Beads are substituted with quaternary amine groups to yield the strong anion exchanger, MonoQ. The stability of the MonoBeads matrix together with controlled synthesis and column packing procedures ensure very reproducible separations both over time and from column to column.

Of course other chromatographic resins, in particular ion-exchange resins, are suitable as well. Examples include Agarose, Sepharose, Sephadex, Sephacryl, Sephacel.

A preferred embodiment of the anion-exchange chromatography, e.g. on MonoQ, is carried out in a buffer of 10 to 50 mM, preferably 15 to 30 mM, in particular 20 mM Tris-HCl, pH 7 to 7.8, preferably 7.3 to 7.6, in particular 7.5 with a linear elution gradient of 0 to 1 M NaCl. Of course, it is possible to use other forms of gradients (e.g. step gradients) or a combination of linear and step gradients, other buffers (e.g. phosphate buffers) and elution salts (e.g. KCI).

As already mentioned above, the fractionated precipitation is carried out by changing the pH and/or ionic strength of the NRL or the extract(s) thereof and/or by introducing a denaturing agent and/or salt thereinto. The addition of salts for fractionated precipitation is most preferred. Suitable salts include ammonium sulphate, NaCl and/or Na₂SO₄. Alternatively, organic solvents such as ethanol or acetone may be used (instead of salts). Referring to the above discussion of protein precipitation ammonium sulphate is the most preferred salt useful in the present invention.

According to a further preferred embodiment of the method of the present invention, the fractionated precipitation is carried out by
(1) adding to the NRL or an extract thereof ammonium sulphate to a concentration of 15 to 35 %, preferably 20 to 30 %, more preferably 22 to 28 %, in particular 25 % saturation,
(2) recovering a first precipitate and a first supernatant by centrifugation,
(3) adding to the first supernatant ammonium sulphate to a concentration of 40 to 60 %, preferably 45 to 55 %, more preferably 47 to 53 %, in particular 50 % saturation,
(4) recovering a second precipitate and a second supernatant by centrifugation,
(5) adding to the second supernatant ammonium sulphate to a concentration of 65 to 85 %, prefarably 70 to 80 %, more preferable 72 to 78 %, in particular 75 % saturation and
(6) recovering a third precipitate and a third supernatant by centrifugation.

The preferred extract of NRL in this case is NAL C-serum. However, the above fractionated precipitation can be applied to any NRL (e.g. NAL) or any other extract derived from NRL.

As a final step, the different protein fraction obtained by fractionated precipitation and/or chromatography may be combined, in order to yield a single composition containing all latex allergens at concentrations sufficient to detect allergen specific IgE.

In particular, the composition(s) obtained by the method defined above contain(s) the allergenic protein of NRL at a higher concentration as the source material (NRL itself or the exctract(s) thereof such as NAL B- or C-serum).

The method of the present invention provides protein compositions which can successfully be used in diagnosis and/or therapy (or for the preparation of a diagnostic and/or pharmaceutical composition (medicament), preferably of latex sensitisation or allergy.

Thus, a further embodiment of the present invention relates to a composition containing substantially all natural human IgE binding proteins of natural rubber latex (NRL) obtainable by the method defined above.

The composition preferably contains protein fractions prepared by
(A) preparation of protein fractions of NAL C-serum by fractionated precipiation,
(B) subjecting the protein fractions obtained in step (A) to anion-exchange chromatography, and
(C) subjecting NAL B-serum to anion-exchange chromatography.

In this respect, it is specifically referred to the explanations and preferred embodiments illustrated above with respect to the method of the present invention.

More preferred, the fractionated precipitation comprises the steps of
(1) adding to NAL C-serum ammonium sulphate to a concentration of 15 to 35 %, preferably 20 to 30 %, more preferably 22 to 28 %, in particular 25 % saturation,
(2) recovering a first precipitate and a first supernatant by centrifugation,
(3) adding to the first supernatant ammonium sulphate to a concentration of 40 to 60 %, preferably 45 to 55 %, more preferably 47 to 53 %, in particular 50 % saturation,
(4) recovering a second precipitate and a second supernatant by centrifugation,
(5) adding to the second supernatant ammonium sulphate to a concentration of 65 to 85 %, preferably 70 to 80 %, more preferable 72 to 78 %, in particular 75 % saturation and
(6) recovering a third precipitate and a third supernatant by centrifugation.

In a further preferred embodiment, the composition contains the following protein fractions (or the composition(s) is/are represented by the following protein fractions):
- the protein fraction obtained by anion-exchange chromatography of the second precipitate in 10 to 50 mM, preferably 15 to 30 mM, in particular 20 mM Tris-HCl, pH 7 to 7.8, preferably 7.3 to 7.6, in particular 7.5, eluting with 120 to 230 mM, preferably 140 to 210 mM, in particular 150 to 200 mM NaCl;
- the protein fraction obtained by anion-exchange chromatography of the second precipitate in 10 to 50 mM, preferably 15 to 30 mM, in particular 20 mM Tris-HCl, pH 7 to 7.8, preferably 7.3 to 7.6, in particular 7.5, eluting with 270 to 530 mM, preferably 290 to 510 mM, in particular 300 to 500 mM NaCl;
- the protein fraction obtained by anion-exchange chromatography of the third precipitate in 10 to 50 mM, preferably 15 to 30 mM, in particular 20 mM Tris-HCl, pH 7 to 7.8, preferably 7.3 to 7.6, in particular 7.5, eluting with 270 to 480 mM, preferably 290 to 460 mM, in particular 300 to 450 mM NaCl;
- the protein fraction obtained by anion-exchange chromatography of the third precipitate in 10 to 50 mM, preferably 15 to 30 mM, in particular 20 mM Tris-HCl, pH 7 to 7.8, preferably 7.3 to 7.6, in particular 7.5, eluting with 420 to 580 mM, preferably 440 to 560 mM, in particular 450 to 550 mM NaCl;
- the protein fraction obtained by anion-exchange chromatography of NAL B-serum in 10 to 50 mM, preferably 15 to 30 mM, in particular 20 mM Tris-HCl, pH 7 to 7.8, preferably 7.3 to 7.6, in particular 7.5, eluting with 120 to 280 mM, preferably 140 to 260 mM, in particular 150 to 250 mM NaCl; and
- the protein fraction obtained by anion-exchange chromatography of NAL B-serum in 10 to 50 mM, preferably 15 to 30 mM, in particular 20 mM Tris-HCl, pH 7 to 7.8, preferably 7.3 to 7.6, in particular 7.5, eluting as the non-adsorbed flow through.

According to a further embodiment of the present invention the composition(s) or protein fraction(s) is/are immobilised on a solid carrier to yield a corresponding carrier-protein conjugate.

It is preferred that the composition is coated onto or coupled to the carrier in the carrier-protein conjugate of the present invention. The carrier may be selected from the group consisting of beads, membranes, dipsticks and glass chips. The conjugate of the present invention are especially useful in diagnostic applications such as the detection of latex allergy or sensitisation in humans.

Therefore, the present invention also relates to a diagnostic kit comprising the composition and/or the conjugate as defined above in combination with commoly used reagents or means for the detection human IgE antibodies.

The diagnostic kit of the present invention may be present in all commonly known forms for the detection of human IgE antibodies. Examples include but are not limited to in vivo tests such as Skin Prick Test that measures an allergic reaction to proteins present in the fractions in the patients' skin, or in vitro tests such as ImmunoCAP assays, RAST assays, ELISA, dipstick, histamine release, etc. that measure the presence of anti latex protein IgE antibodies in the sera of patients tested.

The diagnostic kit is especially useful for the detection of (latex) allergen-specific IgE (in particular in humans).

Accordingly, the present invention further provides a diagnostic method for the detection of (latex) allergen-specific IgE (e.g. in humans) comprising the step of contacting the composition and/or the conjugate of the present invention with a body fluid (e.g. blood or fractions thereof such as blood serum). The contacting with a body fluid also comprises the injection of the composition of the present invention under or into the skin, e.g. by a skin prick text (SPT). The diagnostic method according to the present invention can also be performed as an ImmunoCAP assay, RAST assay, ELISA etc.

The reagents provided by the method of the invention, in particular the composition, are also useful as medicaments (or useful for the preparation thereof). Preferably the inventive composition may be used for the prevention or therapy of latex allergy or latex-fruit syndrome (or for the preparation of a medicament for the mentioned indications). The medicament or pharmaceutical composition can contain commonly used pharmaceutically acceptable one or more carriers, vehicles and/or diluents.

The present invention also relates to a method for the prevention or therapy of the above indications comprising the step of administering the composition or medicament of the present invention to a human patient, particularly suffering from latex allergy or sensitisation and/or fruit allergy. Preferred administration methods include the injection via the intramuscular route or the mucosal application such as via the sublingual route. The preventive/therapeutic method of the present invention is preferably carried out in the form of a hyposensitisation protocol. The protocol may include administration of increasing concentrations of allergen extract over a period of time.

An advantage of the present invention is that comprehensive diagnostics can be carried out using a rather low number of fractions of *Hevea* latex. The present application describes in detail the preparation of these fractions. Contrary thereto, commercial diagnostics of latex allergy are currently carried out using total extracts.

The Figures show:

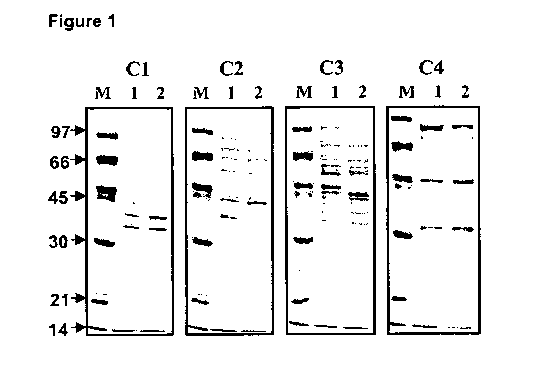

- Figure 1: shows an SDS-PAGE of the four fractions obtained from latex C-serum. Two different batches of latex C-serum were used and protein content compared.

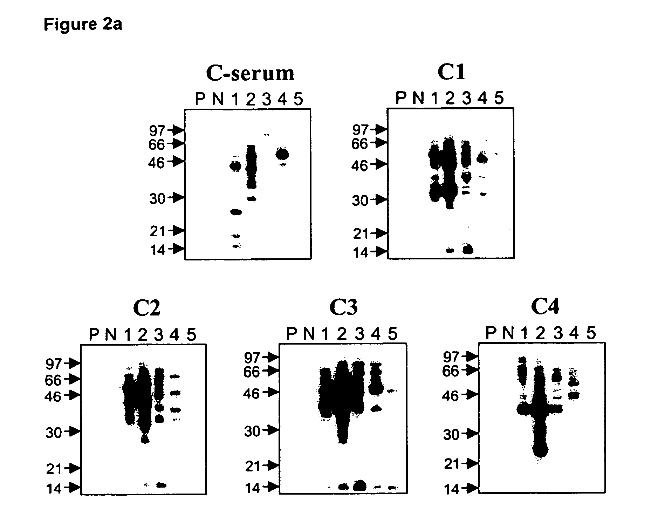

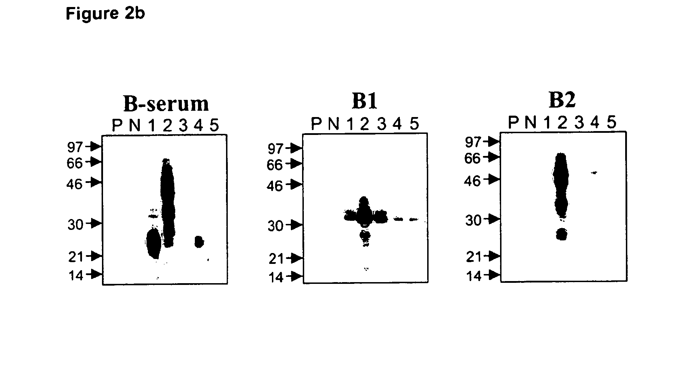

- Figure 2: shows IgE reactivity of 5 serum pools consisting each of 5 sera of latex allergic subjects to a) latex C-serum and the four latex C fractions and b) to latex C-serum and the two latex B fractions. Nitrocellulose-blotted fractions were incubated with the serum pools and bound IgE detected with an ¹²⁵I-labeled anti-human IgE antibody (lanes 1-5). Lanes P and N show the buffer control and the control serum pool. Molecular weight markers as indicated.

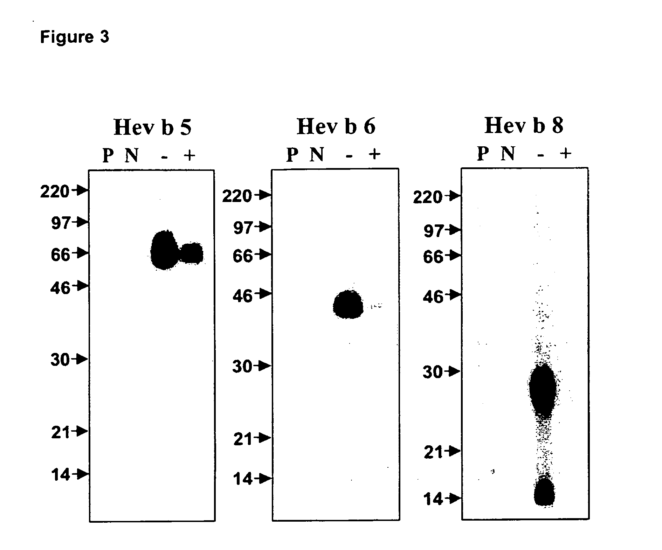

- Figure 3: demonstrates the existence of Hev b 5, Hev b 6, and Hev b 8 in the latex extracts. Nitrocellulose-blotted rHev b 5, rHev b 6, and rHev b 8 were incubated with serum-pools each consisting of three sera of subjects allergic to the respective allergen. The serum pool for Hev b 5 was preincubated with fraction C3, the Hev b 6 serum pool with fraction B2 and the Hev b 8 serum pool with C1. Bound IgE was detected with an ¹²⁵I-labeled anti-human IgE antibody (lanes 1-5). Lanes P and N show the buffer control and the control serum pool. Molecular weight markers as indicated.

The present invention is further illustrated by the following non-limiting examples.

### EXAMPLES

### Example 1: Generation of the latex fractions

### Latex B- and C-serum

Fresh *Hevea* latex was collected in chilled containers from rubber trees (*H*. *brasiliensis,* clone RRIM 600). The latex was centrifuged at 44,000g at 4 °C for 1 hour to separate it into three main fractions: a top fraction containing the rubber particles, an aqueous phase, the C-serum, and a "heavy" bottom fraction containing lutoids. The aqueous phase was collected and centrifugation was repeated. The clear aqueous C-serum was freeze dried and stored at - 20°C. The bottom fraction containing the lutoids was resuspended in 0.4 mol/L mannitol, resedimented and subjected to repeated alternate freezing and thawing to rupture the lutoids. The fluid of the lutoids, the B-serum, was then recovered by centrifugation ¹⁷.

### Fractionating of latex C-serum by ammonium sulphate

One hundred milligrams of lyophilized latex C-serum were dissolved in 20 mM Tris/HCl, pH 7.5. (NH₄)₂SO₄ was added up to 25% saturation and stirred for 30 min at 4 °C. The precipitated material was centrifuged at 12,000g for 20 min at 4°C. The proteins in the supernatant were then precipitated by adding (NH₄)₂SO₄ up to 50% saturation. Precipitated proteins were recovered by centrifugation and (NH₄)₂SO₄ was added to the supernatant up to 75% saturation. The solution was again centrifuged and the proteins from the three precipitation steps stored at -20°C.

### Anion exchange chromatography

Precipitates from the ammonium sulphate fractionation were dissolved in 20 mM Tris/HCl, pH 7.5 and desalted by passing through a PD-10 column (Pharmacia, Uppsala, Sweden). Each fraction was applied to a MonoQ HR5/5 column (Pharmacia). In the same way one hundred milligrams lyophilized latex B-serum were dissolved in 20 mM Tris/HCl, pH 7.5 and applied to a a MonoQ HR5/5 column. The unadsorbed proteins were eluted from the column with the same buffer and adsorbed proteins were eluted using a linear gradient of 0-1 M NaCl in 20 mM Tris/HCl, pH 7.5.

Groups of peaks eluted at different concentrations of NaCl were pooled to fractions C1 - C4. Fraction C1 contained proteins eluted with 150-200 mM NaCl, fraction C2 proteins eluted with 300-500 mM NaCl, both derived from the 50% (NH₄)₂SO₄ precipitate. Fraction C3 contained proteins eluted with 300-450 mM NaCl, C4 with 450-550 mM NaCl, both derived from the 75% (NH₄)₂SO₄ precipitate. The whole procedure was performed with two different batches of latex C-serum. Five µg of each fraction was separated by 12 % PAGE and stained with Coomassie brilliant blue (Figure 1). Protein patterns appeared very similar with small differences in the concentration of certain proteins (Figure 1, lanes 1 and 2).

Latex B-serum was directly applied to anion exchange chromatography yielding two fractions. Fraction B1 contained unadsorbed proteins and fraction B2 proteins eluted with 150-250 mM NaCl.

### Example 2: IgE reactivity to latex C- and B-serum

Ten microgram protein extract or one microgram recombinant protein per lane were separated on 12% SDS/PAGE gels and blotted onto nitrocellulose membranes (Schleicher & Schuell, Dassel, Germany). Membranes were cut into strips and treated with blocking buffer (40 mM Na₂HPO₄, 7 mM NaH₂PO₄, 0.5% BSA, 0.05% w/v sodium azide, 0.5% w/v Tween-20, pH 7.5) for 30 minutes. Strips were then incubated with patients' sera diluted 1 : 5 in blocking buffer overnight at 4°C. After three washing steps blots were incubated with ¹²⁵I-labeled anti-(human IgE) Ig (IBL, Hamburg, Germany) overnight. Patients' IgE binding to the proteins was visualized on Biomax™ MS film (Kodak, New York, USA). A serum pool from 7 healthy atopic individuals with high IgE levels for house dust mite, but negative skin prick tests, and negative CAP results to latex was used as negative control.

IgE reactivity to proteins in latex C-serum was observed for pool 1, 2, and 4 (Figure 2a, blot C-serum). IgE reactions of these serum pools increased in the latex C-serum fractions especially to proteins in the 40 - 80 kDa range (Figure 2a, blots C1-C4, lanes 1, 2, and 4). Serum pool 3 showed weak IgE reactivity to only one protein in latex C-serum (Figure 2a, blot C-serum, lanes 3) and IgE reactions to at least 6 different proteins were measurable in the latex C-serum fractions. No IgE reactions to proteins in latex C-C-serumould be determined for serum pool 5 (Figure 2a, blot C-serum, lane 5) but at least 4 proteins were recognized in the latex C-serum fractions by serum pool 5 (Figure 2a, blots C1-C4, lanes 5). The serum pool of 7 house dust mite allergic subjects and the buffer control showed no IgE reactivity in latex C-serum and any of the fractions (Figure 2a, blots C-serum and C1-C4, lanes N and P).

IgE reactions to proteins in latex B-serum were measurable with serum pools 1, 2, 3, and 4 (Figure 2b, blot B-serum, lanes 1-4). In the latex B-serum fractions no obvious increase in the total number of IgE reactive proteins was detectable with these serum pools but a higher concentration of some proteins especially in the 33 to 35 kDa range was measurable (Figure 2b, blots B1 and B2, lanes 1-4). Serum pool 5 showed no IgE reactivity to latex B-serum whereas a protein band of approximately 33 kDa in fraction B1 was detectable (Figure 2b, blots B-serum, B1, and B2, lanes 5). The serum pool of 7 house dust mite allergic subjects and the buffer control showed no IgE reactivity in latex B-serum and any of the fractions (Figure 2b, blots B-serum, B1, and B2, lanes N and P).

### Example 3: Presence of important latex allergens

The latex fractions of the present invention contain the important latex allergens Hev b 5, Hev b 6, and Hev b 8. Recombinant Hev b 5, rHev b 6, and rHev b 8 were separated by 12% PAGE and blotted onto nitrocellulose. Membrane strips were incubated with serum pools each consisting of three sera tested to display IgE to the respective allergens. For inhibition experiments sera were preincubated with 50 µg protein extract. For inhibition the Hev b 5 serum pool was preincubated with fraction C3, the Hev b 6 serum pool with fraction B2 and the Hev b 8 serum pool with C1. Preincubation of the serum pools with the fractions resulted in diminishment or complete abolishment of IgE binding to the recombinant allergens (Figure 3).

### References

1. Hadjiliadis D, Khan K, Tarlo SM. Skin test responses to latex in an allergy and asthma clinic. J Allergy Clin Immunol 1995; 96:431-2.
2. Hadjiliadis D, Banks DE, Tarlo SM. The relationship between latex skin prick test responses and clinical allergic responses. J Allergy Clin Immunol 1996; 97:1202-6.
3. Hamilton RG, Adkinson NF, Jr. Natural rubber latex skin testing reagents: safety and diagnostic accuracy of nonammoniated latex, ammoniated latex, and latex rubber glove extracts. J Allergy Clin Immunol 1996; 98:872-83.
4. Turjanmaa K, Palosuo T, Alenius H, Leynadier F, Autegarden JE, Andre C, et al. Latex allergy diagnosis: in vivo and in vitro standardization of a natural rubber latex extract. Allergy 1997; 52:41-50.
5. Hamilton RG, Adkinson NF, Jr. Diagnosis of natural rubber latex allergy: multicenter latex skin testing efficacy study. Multicenter Latex Skin Testing Study Task Force. J Allergy Clin Immunol 1998; 102:482-90.
6. Hamilton RG, Peterson EL, Ownby DR. Clinical and laboratory-based methods in the diagnosis of natural rubber latex allergy. J Allergy Clin Immunol 2002; 110:S47-56.
7. Hamilton RG, Biagini RE, Krieg EF. Diagnostic performance of Food and Drug Administration-cleared serologic assays for natural rubber latex-specific IgE antibody. The Multi-Center Latex Skin Testing Study Task Force. J Allergy Clin Immunol 1999; 103:925-30.
8. Ownby DR, Magera B, Williams PB. A blinded, multi-center evaluation of two commercial in vitro tests for latex-specific IgE antibodies. Ann Allergy Asthma Immunol 2000; 84:193-6.
9. Yip L, Hickey V, Wagner B, Liss G, Slater J, Breiteneder H, et al. Skin prick test reactivity to recombinant latex allergens. Int Arch Allergy Immunol 2000; 121:292-9.
10. Bernstein DI, Biagini RE, Kamani R, Hamilton R, Murphy K, Bernstein C, et al. In vivo sensitization to purified Hevea brasiliensis proteins in health care workers sensitized to natural rubber latex. J Allergy Clin Immunol 2003; 111:610-6.
11. Raulf-Heimsoth M, Rihs HP, Bruning T. Latex: a new target for standardization. Arb Paul Ehrlich Inst Bundesamt Sera Impfstoffe Frankf A M 2003:107-15.
12. Yagami T, Osuna H, Kouno M, Haishima Y, Nakamura A, lkezawa Z. Significance of carbohydrate epitopes in a latex allergen with beta-1,3-glucanase activity. Int Arch Allergy Immunol 2002; 129:27-37.
13. Posch A, Chen Z, Dunn MJ, Wheeler CH, Petersen A, Leubner-Metzger G, et al. Latex allergen database. Electrophoresis 1997; 18:2803-10.
14. Arreguin B, Lara P, Rodriguez R. Comparative study of electrophoretic patterns of latex proteins from clones of Hevea brasiliensis. Electrophoresis 1988; 9:323-6.
15. Dennis MS, Light DR. Rubber elongation factor from Hevea brasiliensis. Identification, characterization, and role in rubber biosynthesis. J Biol Chem 1989; 264:18608-17.
16. Tomazic-Jezic VJ, Lucas AD. Protein and allergen assays for natural rubber latex products. J Allergy Clin Immunol 2002; 110:S40-6.
17. Sunderasan E, Hamzah S, Hamid S, Ward MA, Yeang HY, Cardosa MJ. Latex B-B-serum-1,3-glucanase (Hev b 2) and a component of the microhelix (Hev b 4) are major latex allergens. J. Nat. Rubber Res. 1996; 10:82-99.
18. Pridgeon et al. Clin. Exp. Allergy 2000, 301444-1449.
19. Nielson et al. Ann. Allergy Asthma & Immunol. 2000, 85:489-494.

## Claims

1. Method for the production of a composition containing substantially all natural human IgE binding proteins of natural rubber latex (NRL) which comprises the steps of
(a) preparing one or more protein fraction(s) of NRL or an extract thereof by fractionated precipitation and
(b) subjecting the protein fraction(s) obtained in step (a) to chromatographic separation.

2. The method according to claim 1 wherein the NRL is from *Hevea brasiliensis.*

3. The method according to any one of claims 1 to 2 wherein NRL is non-ammoniated latex (NAL).

4. The method according to claim 3 wherein the extract of NAL is obtained by centrifugation.

5. The method according to claim 4 wherein the extract(s) of NAL is/are C-serum and/or B-serum.

6. The method according to claim 5, which comprises the steps of:
(i) preparing one or more protein fraction(s) of NAL C-serum by fractionated precipitation,
(ii) subjecting one or more of the protein fraction(s) obtained in step (a) to chromatographic separation, and
(iii) subjecting NAL B-serum to chromatographic separation.

7. The method according to any one of claims 1 to 6 wherein the fractionated precipitation is carried out by changing the pH and/or ionic strength of the NRL or the extract(s) thereof and/or by introducing a denaturing agent and/or salt thereinto.

8. The method according to claim 7 wherein the fractionated precipitation is carried out by the addition of a salt or an organic solvent.

9. The method according to claim 8 wherein the salt is selected from the group consisting of ammonium sulphate, NaCl and Na₂SO₄.

10. The method according to claim 9, wherein the fractionated precipitation is carried out by
(1) adding to the NRL or an extract thereof ammonium sulphate to a concentration of 15 to 35 %, preferably 20 to 30 %, more preferably 22 to 28 %, in particular 25 % saturation,
(2) recovering a first precipitate and a first supernatant by centrifugation,
(3) adding to the first supernatant ammonium sulphate to a concentration of 40 to 60 %, preferably 45 to 55 %, more preferably 47 to 53 %, in particular 50 % saturation,
(4) recovering a second precipitate and a second supernatant by centrifugation,
(5) adding to the second supernatant ammonium sulphate to a concentration of 65 to 85 %, preferably 70 to 80 %, more preferable 72 to 78 %, in particular 75 % saturation and
(6) recovering a third precipitate and a third supernatant by centrifugation.

11. The method according to claim 10 wherein the extract is NAL C serum.

12. The method according to any one of claims 1 to 11 wherein the chromatographic separation includes ion-exchange chromatography, size exclusion chromatography, hydrophobic chromatography and/or affinity chromatography with latex allergen-specific immunoglobulins.

13. The method according to claim 12 wherein the ion-exchange chromatography is anion-exchange chromatography.

14. The method according to claim 13 wherein anion-exchange chromatography is carried out with a resin selected from the group consisting of MonoQ and DEAE.

15. The method according to claim 14 wherein the anion-exchange chromatography is carried out in a buffer of 10 to 50 mM, preferably 15 to 30 mM, in particular 20 mM Tris-HCl, pH 7 to 7.8, preferably 7.3 to 7.6, in particular 7.5 with a linear elution gradient of 0 to 1 M NaCl.

16. A composition containing substantially all natural human IgE binding proteins of natural rubber latex (NRL) prepared by
(A) preparation of protein fractions of NAL C-serum by fractionated precipiation,
(B) subjecting the protein fractions obtained in step (A) to anion-exchange chromatography, and
(C) subjecting NAL B-serum to anion-exchange chromatography .

17. The composition according to claim 16 wherein the fractionated precipitation comprises the steps of
(1) adding to NAL C-serum ammonium sulphate to a concentration of 15 to 35 %, preferably 20 to 30 %, more preferably 22 to 28 %, in particular 25 % saturation,
(2) recovering a first precipitate and a first supernatant by centrifugation,
(3) adding to the first supernatant ammonium sulphate to a concentration of 40 to 60 %, preferably 45 to 55 %, more preferably 47 to 53 %, in particular 50 % saturation,
(4) recovering a second precipitate and a second supernatant by centrifugation,
(5) adding to the second supernatant ammonium sulphate to a concentration of 65 to 85 %, prefarably 70 to 80 %, more preferable 72 to 78 %, in particular 75 % saturation and
(6) recovering a third precipitate and a third supernatant by centrifugation.

18. The composition according to claim 17 containing
- the protein fraction obtained by anion-exchange chromatography of the second precipitate in 10 to 50 mM, preferably 15 to 30 mM, in particular 20 mM Tris-HCl, pH 7 to 7.8, preferably 7.3 to 7.6, in particular 7.5, eluting with 120 to 230 mM, preferably 140 to 210 mM, in particular 150 to 200 mM NaCl;
- the protein fraction obtained by anion-exchange chromatography of the second precipitate in 10 to 50 mM, preferably 15 to 30 mM, in particular 20 mM Tris-HCl, pH 7 to 7.8, preferably 7.3 to 7.6, in particular 7.5, eluting with 270 to 530 mM, preferably 290 to 510 mM, in particular 300 to 500 mM NaCl;
- the protein fraction obtained by anion-exchange chromatography of the third precipitate in 10 to 50 mM, preferably 15 to 30 mM, in particular 20 mM Tris-HCl, pH 7 to 7.8, preferably 7.3 to 7.6, in particular 7.5, eluting with 270 to 480 mM, preferably 290 to 460 mM, in particular 300 to 450 mM NaCl;
- the protein fraction obtained by anion-exchange chromatography of the third precipitate in 10 to 50 mM, preferably 15 to 30 mM, in particular 20 mM Tris-HCl, pH 7 to 7.8, preferably 7.3 to 7.6, in particular 7.5, eluting with 420 to 580 mM, preferably 440 to 560 mM, in particular 450 to 550 mM NaCl;
- the protein fraction obtained by anion-exchange chromatography of NAL B-serum in 10 to 50 mM, preferably 15 to 30 mM, in particular 20 mM Tris-HCl, pH 7 to 7.8, preferably 7.3 to 7.6, in particular 7.5, eluting with 120 to 280 mM, preferably 140 to 260 mM, in particular 150 to 250 mM NaCl; and
- the protein fraction obtained by anion-exchange chromatography of NAL B-serum in 10 to 50 mM, preferably 15 to 30 mM, in particular 20 mM Tris-HCl, pH 7 to 7.8, preferably 7.3 to 7.6, in particular 7.5, eluting as the non-adsorbed flow through.

19. The composition according to claim 18 wherein the anion-exchange chromatography is carried out on MonoQ or DEAE.

20. Carrier-protein conjugate wherein the composition according to any one of claims 16 to 20 is immobilised on a solid carrier.

21. Conjugate according to claim 20 wherein the carrier is coated with or coupled to the composition.

22. Conjugate according to claim 20 or 21 wherein the carrier is selected from the group consisting of beads, membranes, dipsticks and glass chips.

23. Diagnostic kit comprising the composition according to any one of claims 16 to 19 and/or the conjugate according to any one of claims 20 to 22 in combination with commonly used reagents for the detection human IgE antibodies.

24. Use of the composition according to any one of claims 16 to 19 for the in vitro detection of allergen-specific IgE.

25. Use of the composition according to any one of claims 16 to 19 for the preparation of a medicament for the prevention or therapy of latex allergy or latex-fruit syndrome.

## Patentansprüche

1. Verfahren zum Herstellen einer Zusammensetzung, enthaltend im wesentlichen alle natürlichen, humanes IgE-bindende Proteine von natürlichem Kautschuklatex (NRL), das die Schritte
(a) Herstellen einer oder mehrerer Proteinfraktion(en) von NRL oder einem Extrakt davon durch fraktionierte Ausfällung und
(b) Unterziehen des/der in Schritt (a) erhaltenen Fraktion(en) einer chromatographischen Trennung
umfaßt.

2. Verfahren nach Anspruch 1, wobei der NRL von *Hevea brasiliensis* stammt.

3. Verfahren nach einem der Ansprüche 1 bis 2, wobei NRL nicht mit Ammoniak behandelter Latex (NAL) ist.

4. Verfahren nach Anspruch 3, wobei der Extrakt von NAL durch Zentrifugation erhalten wird.

5. Verfahren nach Anspruch 4, wobei der/die Extrakt(e) von NAL C-Serum und/oder B-Serum ist/sind.

6. Verfahren nach Anspruch 5, das die Schritte:
(i) Herstellen einer oder mehrerer Proteinfraktion(en) von NAL-C-Serum durch fraktionierte Ausfällung,
(ii) Unterziehen einer oder mehrerer in Schritt (a) erhaltener Proteinfraktion(en) einer chromatographischen Trennung und
(iii) Unterziehen des NAL-B-Serums einer chromatographischen Trennung
umfaßt.

7. Verfahren nach einem der Ansprüche 1 bis 6, wobei die fraktionierte Ausfällung durchgeführt wird, indem der pH und/oder die Ionenstärke des NRL oder des Extrakts/der Extrakte davon verändert und/oder ein Denaturierungsmittel und/oder Salz darin eingeführt wird.

8. Verfahren nach Anspruch 7, wobei die fraktionierte Ausfällung durchgeführt wird, indem ein Salz oder ein organisches Lösungsmittel zugegeben wird.

9. Verfahren nach Anspruch 8, wobei das Salz aus der Gruppe ausgewählt ist, bestehend aus Ammoniumsulfat, NaCl und Na₂SO₄.

10. Verfahren nach Anspruch 9, wobei die fraktionierte Ausfällung durchgeführt wird, durch
(1) Zugabe von Ammoniumsulfat zu dem NRL oder einem Extrakt davon auf eine Konzentration von 15 bis 35 %, vorzugsweise 20 bis 30 %, stärker bevorzugt 22 bis 28 %, insbesondere 25 %, Sättigung,
(2) Gewinnen eines ersten Niederschlages und eines ersten Überstandes durch Zentrifugation,
(3) Zugabe von Ammoniumsulfat zu dem ersten Überstand auf eine Konzentration von 40 bis 60 %, vorzugsweise 45 bis 55 %, stärker bevorzugt 47 bis 53 %, insbesondere 50 %, Sättigung,
(4) Gewinnen eines zweiten Niederschlages und eines zweiten Überstandes durch Zentrifugation,
(5) Zugabe von Ammoniumsulfat zu dem zweiten Überstand auf eine Konzentration von 65 bis 85 %, vorzugsweise 70 bis 80 %, stärker bevorzugt 72 bis 78 %, insbesondere 75 %, Sättigung und
(6) Gewinnen eines dritten Niederschlages und eines dritten Überstandes durch Zentrifugation.

11. Verfahren nach Anspruch 10, wobei der Extrakt NAL-C-Serum ist.

12. Verfahren nach einem der Ansprüche 1 bis 11, wobei die chromatographische Trennung Ionenaustauschchromatographie, Größenausschlußchromatographie, hydrophobe Chromatographie und/oder Affinitätschromatographie mit Latexallergen-spezifischen Immunoglobulinen umfaßt.

13. Verfahren nach Anspruch 12, wobei die Ionenaustauschchromatographie Anionenaustauschchromatographie ist.

14. Verfahren nach Anspruch 13, wobei die Anionenaustauschchromatographie mit einem Harz, ausgewählt aus der Gruppe, bestehend aus MonoQ und DEAE, durchgeführt wird.

15. Verfahren nach Anspruch 14, wobei die Anionenaustauschchromatographie in einem Puffer aus 10 bis 50 mM, vorzugsweise 15 bis 30 mM, insbesondere 20 mM, Tris-HCl, pH 7 bis 7,8, vorzugsweise 7,3 bis 7,6, insbesondere 7,5, mit einem linearen Elutionsgradienten von 0 bis 1 M NaCl durchgeführt wird.

16. Zusammensetzung, enthaltend im wesentlichen alle natürlichen, humanes IgE-bindende Proteine von natürlichem Kautschuklatex (NRL), hergestellt durch
(A) Herstellen von Proteinfraktionen von NAL-C-Serum durch fraktionierte Ausfällung,
(B) Unterziehen der in Schritt (A) erhaltenen Proteinfraktionen einer Anionenaustauschchromatographie, und
(C) Unterziehen des NAL-B-Serums einer Anionenaustauschchromatographie.

17. Zusammensetzung nach Anspruch 16, wobei die fraktionierte Ausfällung die Schritte
(1) Zugeben von Ammoniumsulfat zu NAL-C-Serum auf eine Konzentration von 15 bis 35 %, vorzugsweise 20 bis 30 %, stärker bevorzugt 22 bis 28 %, insbesondere 25 %, Sättigung,
(2) Gewinnen eines ersten Niederschlages und eines ersten Überstandes durch Zentrifugation,
(3) Zugeben von Ammoniumsulfat zu dem ersten Überstand auf eine Konzentration von 40 bis 60 %, vorzugsweise 45 bis 55 %, stärker bevorzugt 47 bis 53 %, insbesondere 50 %, Sättigung,
(4) Gewinnen eines zweiten Niederschlages und eines zweiten Überstandes durch Zentrifugation,
(5) Zugeben von Ammoniumsulfat zu dem zweiten Überstand auf eine Konzentration von 65 bis 85 %, vorzugsweise 70 bis 80 %, stärker bevorzugt 72 bis 78 %, insbesondere 75 %, Sättigung und
(6) Gewinnen eines dritten Niederschlages und eines dritten Überstandes durch Zentrifugation
umfaßt.

18. Zusammensetzung nach Anspruch 17, enthaltend
- die Proteinfraktion, erhalten durch Anionenaustauschchromatographie des zweiten Niederschlages in 10 bis 50 mM, vorzugsweise 15 bis 30 mM, insbesondere 20 mM, Tris-HCl, pH 7 bis 7,8, vorzugsweise 7,3 bis 7,6, insbesondere 7,5, unter Elution mit 120 bis 230 mM, vorzugsweise 140 bis 210 mM, insbesondere 150 bis 200 mM, NaCl;
- die Proteinfraktion, erhalten durch Anionenaustauschchromatographie des zweiten Niederschlages in 10 bis 50 mM, vorzugsweise 15 bis 30 mM, insbesondere 20 mM, Tris-HCl, pH 7 bis 7,8, vorzugsweise 7,3 bis 7,6, insbesondere 7,5, unter Elution mit 270 bis 530 mM, vorzugsweise 290 bis 510 mM, insbesondere 300 bis 500 mM, NaCl;
- die Proteinfraktion, erhalten durch Anionenaustauschchromatographie des dritten Niederschlages in 10 bis 50 mM, vorzugsweise 15 bis 30 mM, insbesondere 20 mM, Tris-HCl, pH 7 bis 7,8, vorzugsweise 7,3 bis 7,6, insbesondere 7,5, unter Elution mit 270 bis 480 mM, vorzugsweise 290 bis 460 mM, insbesondere 300 bis 450 mM, NaCl;
- die Proteinfraktion, erhalten durch Anionenaustauschchromatographie des dritten Niederschlages in 10 bis 50 mM, vorzugsweise 15 bis 30 mM, insbesondere 20 mM, Tris-HCl, pH 7 bis 7,8, vorzugsweise 7,3 bis 7,6, insbesondere 7,5, unter Elution mit 420 bis 580 mM, vorzugsweise 440 bis 560 mM, insbesondere 450 bis 550 mM, NaCl;
- die Proteinfraktion, erhalten durch Anionenaustauschchromatographie von NAL-B-Serum in 10 bis 50 mM, vorzugsweise 15 bis 30 mM, insbesondere 20 mM, Tris-HCl, pH 7 bis 7,8, vorzugsweise 7,3 bis 7,6, insbesondere 7,5, unter Elution mit 120 bis 280 mM, vorzugsweise 140 bis 260 mM, insbesondere 150 bis 250 mM, NaCl; und
- die Proteinfraktion, erhalten durch Anionenaustauschchromatographie von NAL-B-Serum in 10 bis 50 mM, vorzugsweise 15 bis 30 mM, insbesondere 20 mM, Tris-HCl, pH 7 bis 7,8, vorzugsweise 7,3 bis 7,6, insbesondere 7,5, unter Elution als der nicht-adsorbierte Durchfluß.

19. Zusammensetzung nach Anspruch 18, wobei die Anionenaustauschchromatographie an MonoQ oder DEAE durchgeführt wird.

20. Träger-Protein-Konjugat, wobei die Zusammensetzung nach einem der Ansprüche 16 bis 20 auf einem festen Träger immobilisiert ist.

21. Konjugat nach Anspruch 20, wobei der Träger mit der Zusammensetzung beschichtet oder damit verbunden ist.

22. Konjugat nach Anspruch 20 oder 21, wobei der Träger aus der Gruppe ausgewählt ist, bestehend aus Kügelchen, Membranen, Meßstäben und Glassplittern.

23. Diagnostisches Kit, umfassend die Zusammensetzung nach einem der Ansprüche 16 bis 19 und/oder das Konjugat nach einem der Ansprüche 20 bis 22 in Kombination mit üblicherweise verwendeten Reagenzien für die Detektion menschlicher IgE-Antiköper.

24. Verwendung der Zusammensetzung nach einem der Ansprüche 16 bis 19 für die In-Vitro-Detektion von Allergen-spezifischem IgE.

25. Verwendung der Zusammensetzung nach einem der Ansprüche 16 bis 19 zur Herstellung eines Medikaments zur Vorbeugung oder Therapie von Latexallergie oder Latex-Frucht-Syndrom.

## Revendications

1. Procédé pour la production d'une composition contenant essentiellement toutes les protéines naturelles de liaison d'IgE humains du latex de caoutchouc naturel (NRL) qui comprend les étapes de :
(a) préparation d'une ou plusieurs fractions de protéine de NRL ou d'un extrait de celui-ci par précipitation fractionnée et
(b) traitement de la ou des fractions de protéine obtenues dans l'étape (a) en les soumettant à une séparation chromatographique.

2. Procédé suivant la revendication 1, dans lequel le NRL provient d*'Hevea brasiliensis.*

3. Procédé suivant l'une ou l'autre des revendications 1 et 2, dans lequel le NRL est du latex non ammoniacé (NAL).

4. Procédé suivant la revendication 3, dans lequel l'extrait de NAL est obtenu par centrifugation.

5. Procédé suivant la revendication 4, dans lequel l'extrait ou les extraits de NAL est/sont du sérum C et/ou du sérum B.

6. Procédé suivant la revendication 5, qui comprend les étapes de :
(i) préparation d'une ou plusieurs fractions de protéine de sérum C de NAL par précipitation fractionnée,
(ii) traitement d'une ou plusieurs des fractions de protéine obtenues dans l'étape (a) en les soumettant à une séparation chromatographique, et
(iii) traitement du sérum B de NAL en le soumettant à une séparation chromatographique.

7. Procédé suivant l'une quelconque des revendications 1 à 6, dans lequel la précipitation fractionnée est réalisée en changeant le pH et/ou la force ionique du NRL ou de son ou ses extraits et/ou en y introduisant un agent dénaturant et/ou un sel.

8. Procédé suivant la revendication 7, dans lequel la précipitation fractionnée est réalisée par l'addition d'un sel ou d'un solvant organique.

9. Procédé suivant la revendication 8, dans lequel le sel est choisi dans le groupe comprenant le sulfate d'ammonium, NaCl et Na₂SO₄.

10. Procédé suivant la revendication 9, dans lequel la précipitation fractionnée est réalisée en :
(1) ajoutant au NRL ou à un extrait de celui-ci du sulfate d'ammonium à une concentration de 15 à 35 %, avantageusement 20 à 30 %, plus avantageusement 22 à 28 %, en particulier 25 % de la saturation,
(2) en récupérant un premier précipité et un premier surnageant par centrifugation,
(3) en ajoutant au premier surnageant du sulfate d'ammonium à une concentration de 40 à 60 %, avantageusement 45 à 55 %, plus avantageusement 47 à 53 %, en particulier 50 % de la saturation,
(4) en récupérant un second précipité et un second surnageant par centrifugation,
(5) en ajoutant au second surnageant du sulfate d'ammonium à une concentration de 65 à 85 %, avantageusement 70 à 80 %, plus avantageusement 72 à 78 %, en particulier 75 % de la saturation, et
(6) en récupérant un troisième précipité et un troisième surnageant par centrifugation.

11. Procédé suivant la revendication 10, dans lequel l'extrait est du sérum C de NAL.

12. Procédé suivant l'une quelconque des revendications 1 à 11, dans lequel la séparation chromatographique comprend une chromatographie d'échange d'ions, une chromatographie d'exclusion en fonction de la taille, une chromatographie hydrophobe et/ou une chromatographie d'affinité avec des immunoglobulines spécifiques d'allergènes de latex.

13. Procédé suivant la revendication 12, dans lequel la chromatographie d'échange d'ions est une chromatographie d'échange d'anions.

14. Procédé suivant la revendication 13, dans lequel la chromatographie d'échange d'anions est réalisée avec une résine choisie dans le groupe comprenant MonoQ et DEAE.

15. Procédé suivant la revendication 14, dans lequel la chromatographie d'échange d'anions est réalisée dans un tampon de Tris-HCI 10 à 50 mM, avantageusement 15 à 30 mM, en particulier 20 mM, à un pH de 7 à 7,8, avantageusement 7,3 à 7,6, en particulier 7,5 avec un gradient d'élution linéaire de NaCl 0 à 1 M.

16. Composition contenant essentiellement toutes les protéines naturelles de liaison d'IgE humains du latex de caoutchouc naturel (NRL) préparée par :
(A) préparation de fractions de protéine de sérum C de NAL par précipitation fractionnée,
(B) traitement des fractions de protéine obtenues dans l'étape (A) en les soumettant à une chromatographie d'échange d'anions, et
(C) traitement du sérum B de NAL en le soumettant à une chromatographie d'échange d'anions.

17. Composition suivant la revendication 16, dans lequel la précipitation fractionnée comprend les étapes :
(1) d'addition au sérum C de NAL de sulfate d'ammonium à une concentration de 15 à 35%, avantageusement 20 à 30 %, plus avantageusement 22 à 28 %, en particulier 25 % de la saturation,
(2) de récupération d'un premier précipité et d'un premier surnageant par centrifugation,
(3) d'addition au premier surnageant de sulfate d'ammonium à une concentration de 40 à 60 %, avantageusement 45 à 55 %, plus avantageusement 47 à 53 %, en particulier 50 % de la saturation,
(4) de récupération d'un second précipité et d' un second surnageant par centrifugation,
(5) d'addition au second surnageant de sulfate d'ammonium à une concentration de 65 à 85 %, avantageusement 70 à 80 %, plus avantageusement 72 à 78 %, en particulier 75 % de la saturation, et
(6) de récupération d'un troisième précipité et d'un troisième surnageant par centrifugation.

18. Composition suivant la revendication 17, contenant :
- la fraction de protéine obtenue par chromatographie d'échange d'anions du second précipité dans du Tris-HCI 10 à 50 mM, avantageusement 15 à 30 mM, en particulier 20 mM, à un pH de 7 à 7,8, avantageusement de 7,3 à 7,6, en particulier de 7,5, en éluant avec du NaCl 120 à 230 mM, avantageusement 140 à 210 mM, en particulier 150 à 200 mM;
- la fraction de protéine obtenue par chromatographie d'échange d'anions du second précipité dans du Tris-HCl 10 à 50 mM, avantageusement 15 à 30 mM, en particulier 20 mM, à un pH de 7 à 7,8, avantageusement de 7,3 à 7,6, en particulier de 7,5, en éluant avec du NaCl 270 à 530 mM, avantageusement 290 à 510 mM, en particulier 300 à 500 mM;
- la fraction de protéine obtenue par chromatographie d'échange d'anions du troisième précipité dans du Tris-HCl 10 à 50 mM, avantageusement 15 à 30 mM, en particulier 20 mM, à un pH de 7 à 7,8, avantageusement de 7,3 à 7,6, en particulier de 7,5, en éluant avec du NaCl 270 à 480 mM, avantageusement 290 à 460 mM, en particulier 300 à 450 mM;
- la fraction de protéine obtenue par chromatographie d'échange d'anions du troisième précipité dans du Tris-HCl 10 à 50 mM, avantageusement 15 à 30 mM, en particulier 20 mM, à un pH de 7 à 7,8, avantageusement de 7,3 à 7,6, en particulier de 7,5, en éluant avec du NaCl 420 à 580 mM, avantageusement 440 à 560 mM, en particulier 450 à 550 mM;
- la fraction de protéine obtenue par chromatographie d'échange d'anions de sérum B de NAL dans du Tris-HCl 10 à 50 mM, avantageusement 15 à 30 mM, en particulier 20 mM, à un pH de 7 à 7,8, avantageusement de 7,3 à 7,6, en particulier de 7,5, en éluant avec du NaCl 120 à 280 mM, avantageusement 140 à 260 mM, en particulier 150 à 250 mM; et
- la fraction de protéine obtenue par chromatographie d'échange d'anions de sérum B de NAL dans du Tris-HCl 10 à 50 mM, avantageusement 15 à 30 mM, en particulier 20 mM, à un pH de 7 à 7,8, avantageusement de 7,3 à 7,6, en particulier de 7,5, en éluant comme écoulement de passage non adsorbé.

19. Composition suivant la revendication 18, dans laquelle la chromatographie d'échange d'anions est réalisée sur MonoQ ou DEAE.

20. Produit conjugué support-protéine dans lequel la composition suivant l'une quelconque des revendications 16 à 20 est immobilisée sur un support solide.

21. Produit conjugué suivant la revendication 20, dans lequel le support est revêtu de la composition ou couplé à celle-ci.

22. Produit conjugué suivant l'une ou l'autre des revendications 20 et 21, dans lequel le support est choisi dans le groupe comprenant les perles, les membranes, les bandelettes et les fragments de verre.

23. Trousse de diagnostic comprenant la composition suivant l'une quelconque des revendications 16 à 19 et/ou le produit conjugué suivant l'une quelconque des revendications 20 à 22 en combinaison à des réactifs communément utilisés pour la détection d'anticorps IgE humains.

24. Utilisation de la composition suivant l'une quelconque des revendications 16 à 19 pour la détection in vitro d'IgE spécifiques d'allergènes.

25. Utilisation de la composition suivant l'une quelconque des revendications 16 à 19 pour la préparation d'un médicament pour la prévention ou la thérapie d'une allergie au latex ou d'un syndrome aux fruits du latex.
